(19) Europäisches Patentamt
European Patent Office
Office européen des brevets

(11) EP 0 449 288 B1

(12) EUROPÄISCHE PATENTSCHRIFT

(45) Veröffentlichungstag und Bekanntmachung des Hinweises auf die Patenterteilung:
**11.09.1996 Patentblatt 1996/37**

(51) Int. Cl.[6]: **C07C 25/18**, C09K 19/14, C07C 43/225, C09K 19/30, G02F 1/13

(21) Anmeldenummer: **91105001.1**

(22) Anmeldetag: **27.03.1991**

(54) **Phenylethane und flüssigkristallines Medium**
Phenylethanes and liquid crystal medium
Phényléthanes et médium cristallin liquide

(84) Benannte Vertragsstaaten:
**DE GB**

(30) Priorität: **28.03.1990 DE 4009929**

(43) Veröffentlichungstag der Anmeldung:
**02.10.1991 Patentblatt 1991/40**

(73) Patentinhaber: **MERCK PATENT GmbH**
**D-64271 Darmstadt (DE)**

(72) Erfinder:
- **Hittich, Reinhard, Dr.**
**W-6101 Modautal 1 (DE)**
- **Krause, Joachim, Dr.**
**W-6110 Dieburg (DE)**
- **Poetsch, Eike, Dr.**
**D-6109 Mühltal (DE)**
- **Plach, Herbert, Dr.**
**D-6100 Darmstadt (DE)**

(56) Entgegenhaltungen:
**WO-A-85/04874** **WO-A-90/08757**
**WO-A-91/05029**

- **PROCEEDINGS OF THE SPIE THE INTERNATIONAL SOCIE- TY FOR OPTICAL ENGINEERING, vol.1257,24 April 19 90,BELLINGHAM,WASHINGTON,USA,Seite 84-94 V.REIFFENRATH: "Liquid crystalline materials"**
- **CHEMICAL ABSTRACTS, vol. 116, no. 8, 24. Februar 1992, Columbus, Ohio, US; abstract no. 72519, Seite 898 ; & JP-A-03 206 055**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 449 288 B1

Printed by Rank Xerox (UK) Business Services
2.13.4/3.4

## Beschreibung

Die Erfindung betrifft neue Phenylethane der Formel I

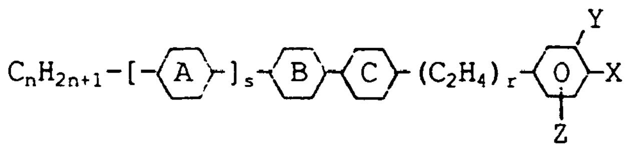

I

worin n 1 bis 7, r 1 oder 2, die Ringe A, B und C jeweils unabhängig voneinander trans-1,4-Cyclohexylen, 1,4-Phenylen oder 3-Fluor-1,4-Phenylen, Ring C auch 3,5-Difluor-1,4-phenylen, s 1 oder im Falle C = 3,5-Difluor-1,4-phenylen oder B = 3-Fluor-1,4-phenylen auch 0, X F, Cl, $-CF_3$, $-OCF_3$ oder $-OCHF_2$ und Y und Z jeweils unabhängig voneinander H oder F bedeuten, mit der Maßgabe, daß im Falle

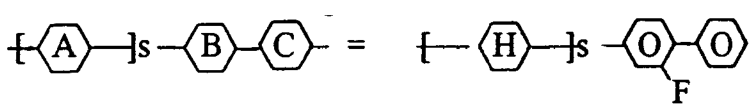

und r = 1, Y = Z = F bedeutet.

Aus der DE-OS 37 34 116 sind Flüssigkristalle der Formel A

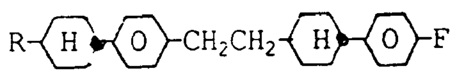

A

bekannt, worin R Alkyl bedeutet.

Die Verbindungen A zeichnen sich jedoch nur durch eine moderate dielektrische Anisotropie aus, die in Mischungen oft zu relativ hohen Schwellenspannungen führt.

Die Verbindungen der Formel I können wie ähnliche, z.B. aus der DE-OS 33 17 597 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung.

Insbesondere bei Anzeigen vom Supertwisttyp (STN) mit Verdrillungswinkeln von deutlich mehr als 220 °C oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hohem Klärpunkt, hervorragender Nematogenität bis zu tiefen Temperaturen, hervorragender chemischer Stabilität, ausgeprägtem $\varepsilon\perp$ bei positiver dieelektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben n, r, s, A, B, C, X, Y und Z die angegebene Bedeutung, sofern nicht ausdrücklich etwas anderes vermerkt ist.

In den Verbindungen der Formel I sind die Alkylgruppen $C_nH_{2n+1}$ vorzugsweise geradkettig. Dementsprechend bedeutet $C_nH_{2n+1}$ Methyl, Ethyl, n-Propyl, n-Butyl, n-Pentyl, n-Hexyl oder n-Heptyl. n ist vorzugsweise 2, 3, 4 oder 5.

Verbindungen der Formel I mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methyl-propyl), Isobutyl (= 2-Methyl-propyl), 2-Methylbutyl, Isopentyl (= 3-Methylbutyl), 2-Methylpentyl, 3-Methylpentyl oder 2-Heptyl (= 1-Methylhexyl).

Der Rest

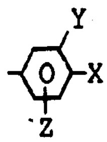

ist vorzugsweise

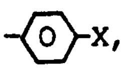, 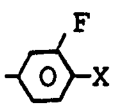 oder 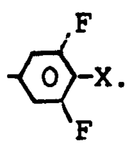.

X ist vorzugsweise F, Cl, $-CF_3$ oder $-OCF_3$.

Besonders bevorzugt sind die Verbindungen der folgenden Teilformeln:

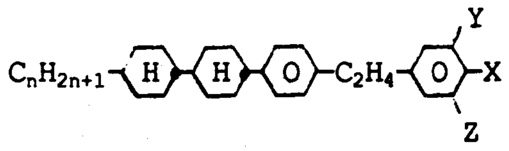

Ia

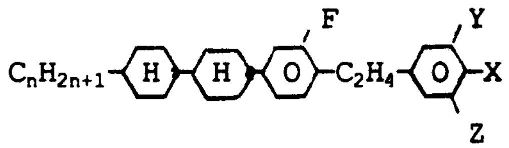

Ib

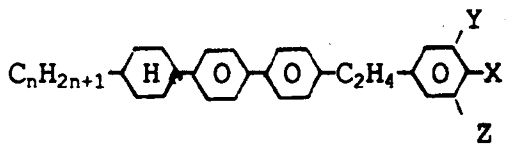

Ic

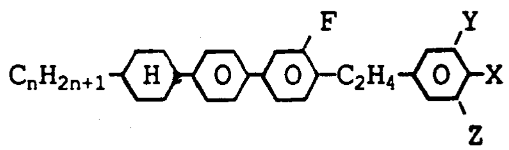

Id

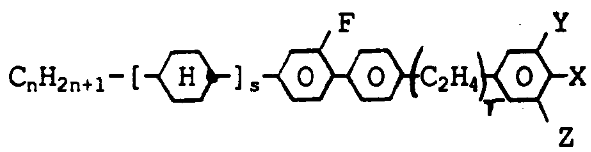

Ie

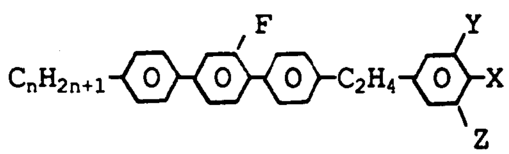

If

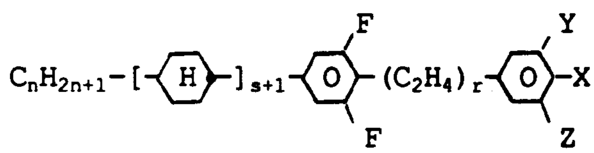

Ig

Von den Verbindungen der bevorzugten Teilformeln Ia, Ie und Ig sind diejenigen besonders bevorzugt, worin Y = F und insbesondere diejenigen, worin Y = Z = F bedeutet.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I können beispielsweise durch Umsetzung eines Aldehyds der Formel

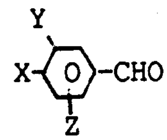

mit einem Phosphoniumsalz der Formel II

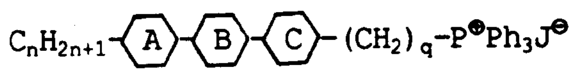

II

nach Wittig und katalytischer Hydrierung z.B. an Pd/C der erhaltenen Ethenderivate erhalten werden.

In Formel II bedeutet q 1 oder 3.

Die Verbindungen der Formel II sind erhältlich durch Verseifung der bekannten Nitrile der Formel

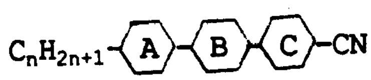

Reduktion zum Methylalkohol und Umwandlung in die entsprechenden Iodide nach Routinemethoden. Die Verbindungen mit q = 3 können hergestellt werden, indem man die Iodide einer Kettenverlängerung nach Routinemethoden ($-CH_2J \rightarrow CH_2CN \rightarrow CH_2COOH \rightarrow CH_2CH_2OH \rightarrow (CH_2)_2J$ usw.) unterwirft.

Die als Ausgangsstoffe verwendeten Brombenzolderivate (Schema 1) sind zum Teil bekannt, zum Teil können sie ohne Schwierigkeiten nach Standardverfahren der organischen Chemie aus literaturbekannten Verbindungen hergestellt werden. Beispielsweise sind die $OCF_3$- oder $OCHF_2$-Verbindungen nach bekannten Verfahren aus den entsprechenden Phenolen bzw. die $CF_3$- oder CN-Verbindungen aus den entsprechenden Benzoesäuren erhältlich. Verbindungen der Formel

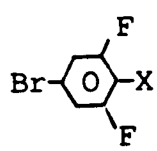

oder auch entsprechende monofluorierte Verbindungen sind beispielsweise aus den bekannten Vorstufen mit X = H durch Lithiierung bei tiefen Temperaturen und anschließende Umsetzung mit einem geeigneten Elektrophil erhältlich.

Weitere Synthesevarianten sind dem Fachmann bekannt. Bevorzugte Varianten sind dem Schema 1 zu entnehmen. Alle Ausgangsmaterialien sind entweder bekannt oder können in Analogie zu bekannten Verbindungen hergestellt werden.

Schema 1

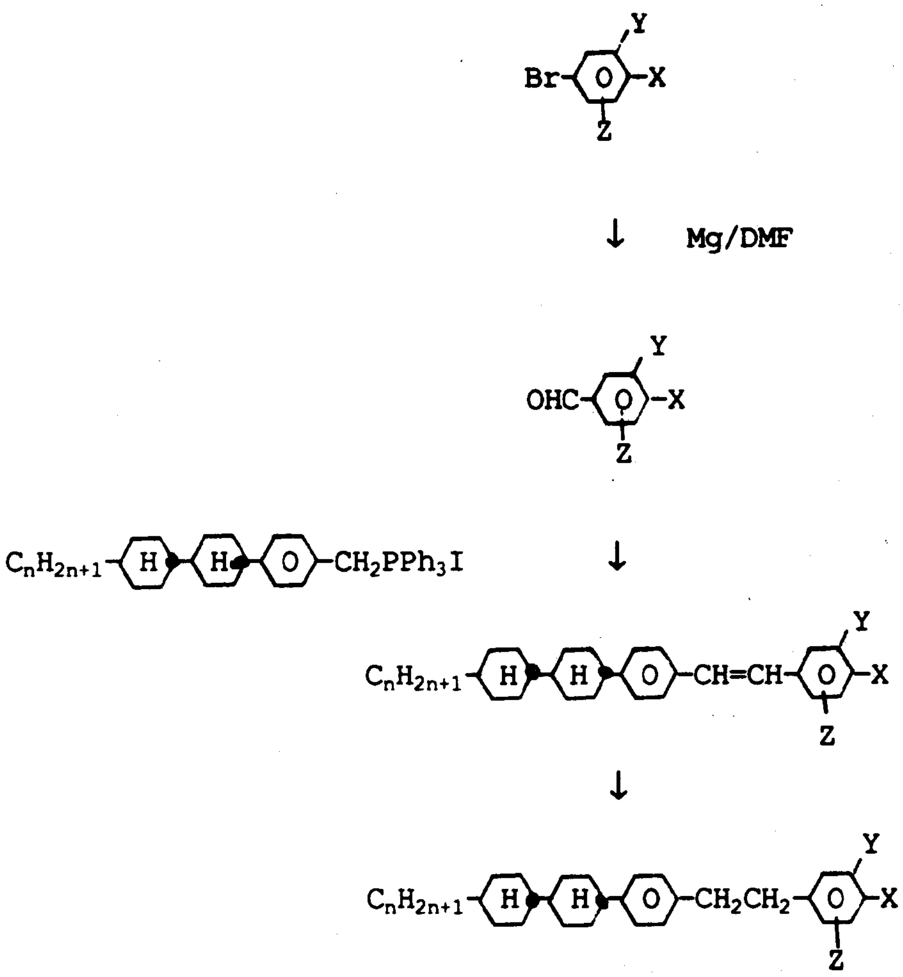

**Schema 2**

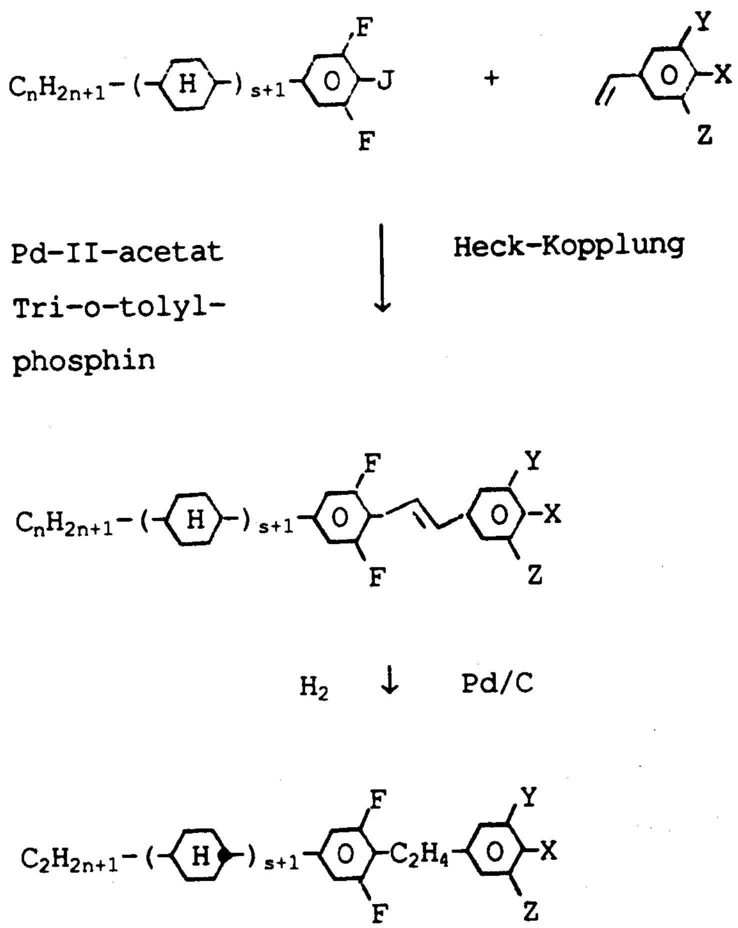


Die Styrolvorstufe ihrerseits ist ebenfalls leicht aus einem entsprechenden Brom- oder Iodbenzolderivat nach Heck- bzw. Pd-katalysierter Kopplung der zinkorganischen Verbindung mit Vinylbromid erhältlich.

Erfindungsgemäße Verbindungen mit Y = F können z.B. hergestellt werden, indem man eine Verbindung der Formel II,

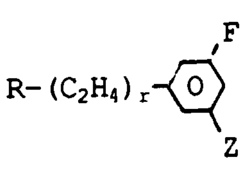

II

worin R,

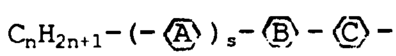

bedeutet, gemäß folgendem Reaktionsschema metalliert und anschließend mit einem geeigneten Elektrophil umsetzt:

Schema 1

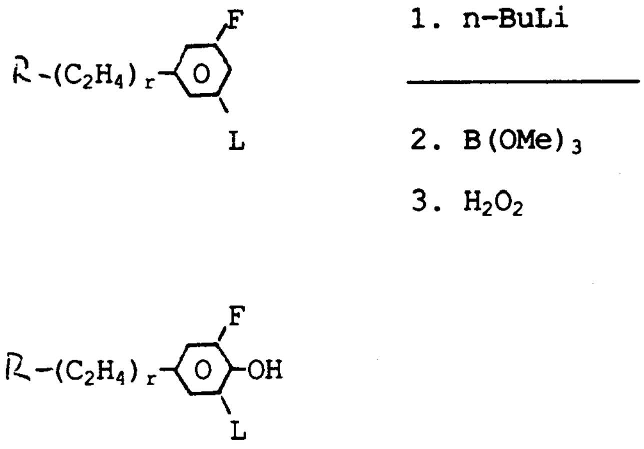


Aus dem erhaltenen Phenol sind die Zielprodukte mit X = $OCF_3$ oder $OCHF_2$ nach bekannten Methoden, z. B. durch Umsetzung mit Chlordifluormethan bzw. Tetrachlorkohlenstoff/HF erhältlich.

Weitere Synthesemethoden sind für den Fachmann augenscheinlich. Beispielsweise können in 5-Position entsprechend substituierte 1,3-Difluorbenzol-Verbindungen oder monofluorierte Analoga (Z = H) gemäß obigem Schema in die 2-$OCF_2Y'$-1,3-difluor-Verbindungen (Y' = H oder F) oder monofluorierte Analoga (Z = F) überführt werden und der Rest R-$(C_2H_4)_r$ anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) eingeführt werden.

Schema 2

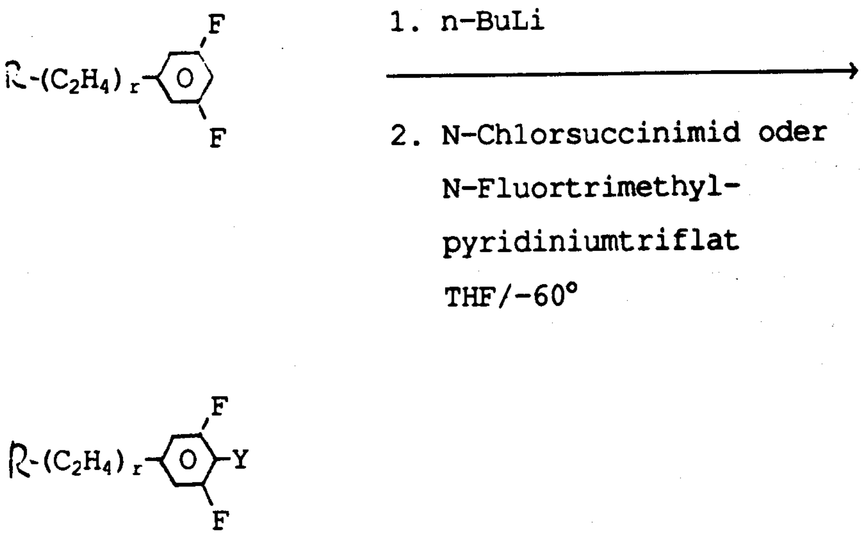


Die erfindungsgemäßen Verbindungen der Formel I, worin Z F und X $CF_3$ bedeutet, lassen sich durch Metallierung der unsubstituierten 3,5-Difluorphenylverbindungen mit n-BuLi, anschließender Reaktion mit Jod und Umsetzung der Jodverbindung mit Trifluoressigsäure-Natriumsalz gemäß folgenden Schema herstellen:

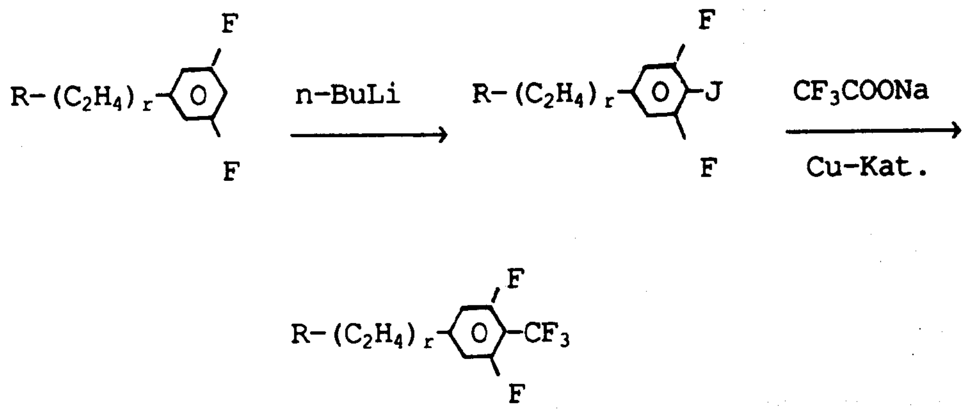


Die erfindungsgemäßen Verbindungen mit Z = H und X = $CF_3$ können hergestellt werden, indem man 3-Fluor-4-jodbrombenzol mit $CF_3COONa$ in die Benzotrifluoridverbindung überführt und anschließend den Rest R-$(C_2H_4)_r$ z.B. über übliche Kopplungsreaktionen einführt.

Die Verbindungen der Formel II können beispielsweise nach folgenden Syntheseschemata hergestellt werden:

<u>Schema 3</u> (A = $(C_2H_4)_{r-1}$)

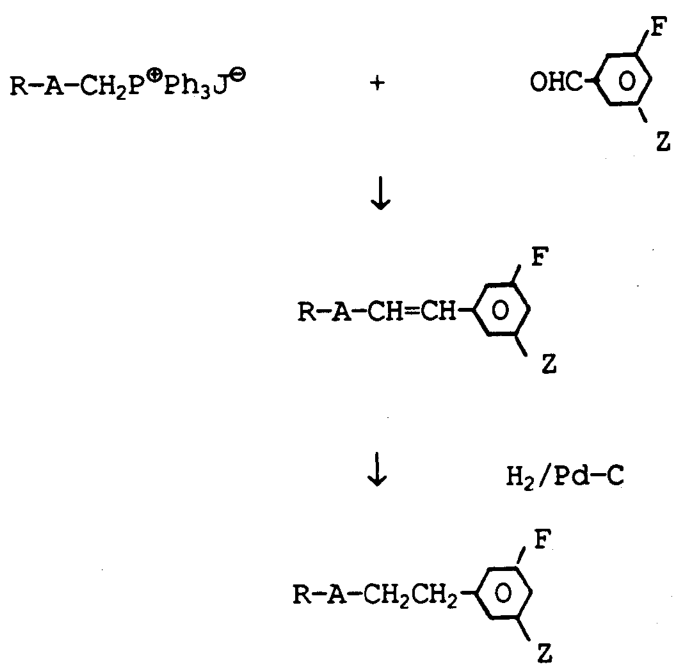


Die Ausgangsmaterialien sind entweder bekannt oder können in Analogie zu bekannten Verbindungen hergestellt werden.

Die Synthese einiger besonders bevorzugter Verbindungen ist im folgenden näher angegeben:

Schema 4

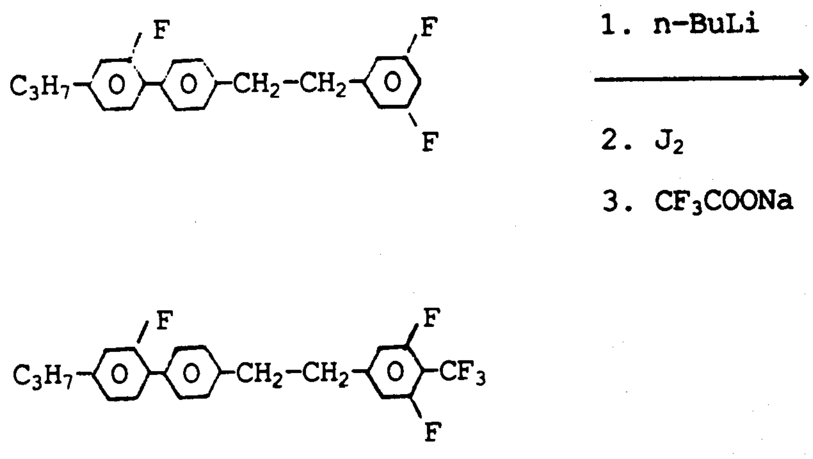


In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146). Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere (Pd(II)-acetat, mit organischen Phosphor(III)-Verbindungen wie z.B. Triarylphosphanen geeignet. Man kann dabei in Gegenwart oder Abwesenheit eines inerten Lösungsmittels bei Temperaturen zwischen etwa 0° und 150°, vorzugsweise zwischen 20° und 100°, arbeiten; als Lösungsmittel kommen z.B. Nitrile wie Acetonitrile oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-substituierten Benzaldehyds mit einem entsprechenden Phoshorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinnverbindungen umgesetzt werden. Bevorzugt werden diese Reaktionen unter Zusatz eines Katalysators wie z.B. eines Palladium(0)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J. Org. Chem. 43, 358 (1978) beschriebenen Verfahren durchgeführt werden. Katalytische Hydrierungen dieser Materialien führen zu den erfindungsgemäßen Verbindungen.

Die Verbindungen der Formel I mit r = Z können nach folgendem Schema hergestellt werden:

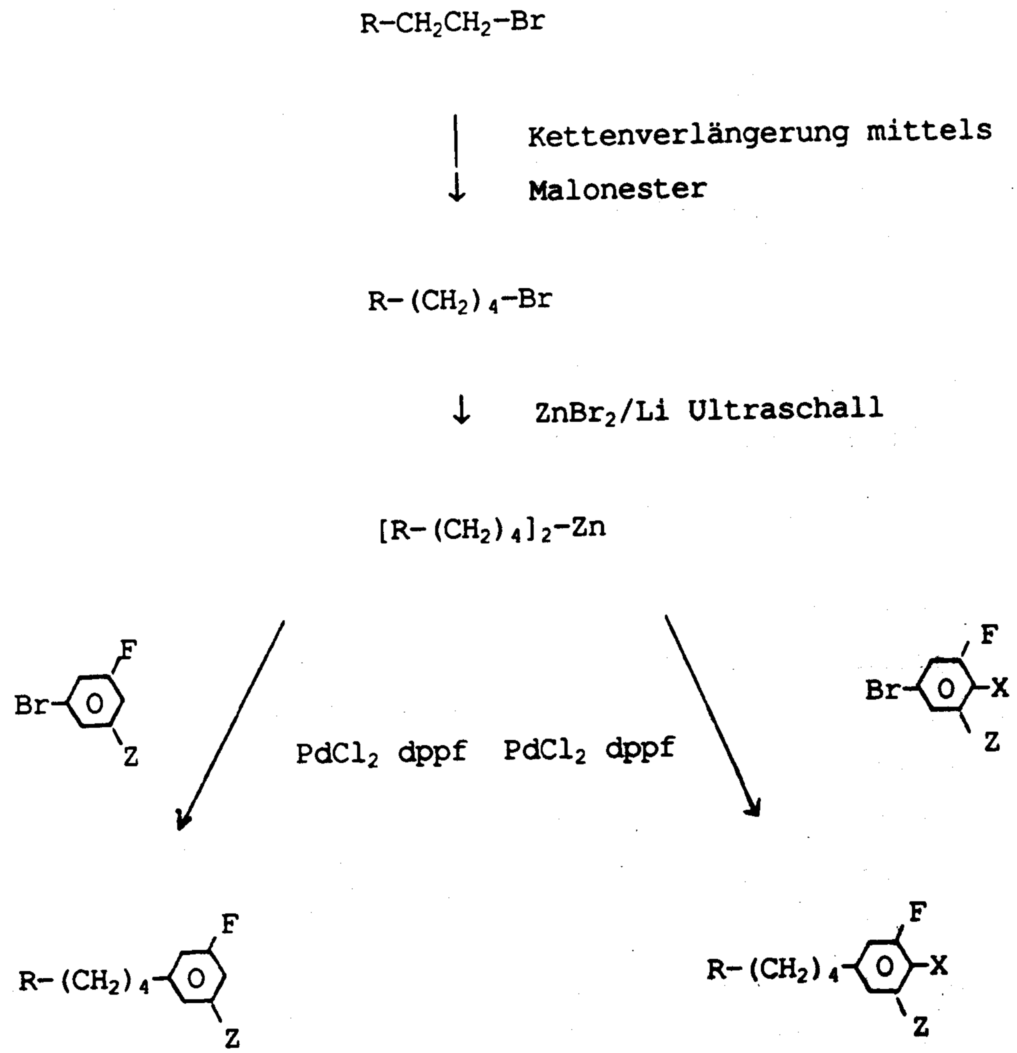


Bei der Pd(II)-katalysierten Kopplungsreaktion wird entweder direkt das Zielprodukt I gebildet oder ein Vorprodukt, in das völlig analog zu den vorstehenden Methoden für Verbindungen oder Formel I der Rest X eingeführt wird.

Die Verbindungen der Formel I können ferner nach Wittig gemäß folgendem Schema hergestellt werden:

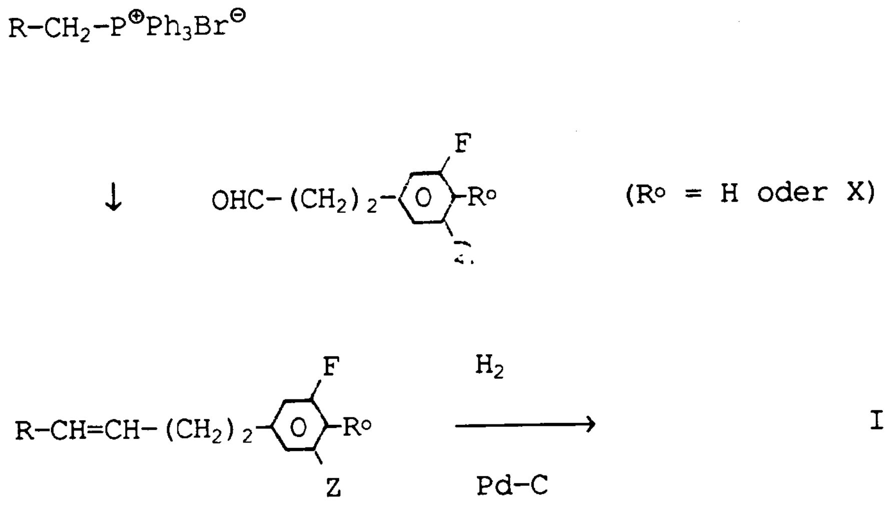


Die bevorzugten trans-Isomeren können nach können nach den literaturbekannten Isomerisierungsmethoden hergestellt werden. Die ggf. erhaltenen Vorprodukte mit $R_o$ = H werden völlig analog zu den Vorprodukten der Verbindungen der Formel I durch Einführen des Restes X in die Verbindungen der Formel I übergeführt.

Die Aldehyde können durch Heck-Reaktion von entsprechend substituierten 1-Brom-3-fluorbenzolderivaten mit Allylalkohol erhalten werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl-oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

$R'-L-CH_2CH_2-E-R''$ 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio

1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, $-CF_3$, $-OCF_3$, F, Cl oder -NCS; R hat dabei die bei den Verbindungen der Teilformeln 1a bis 5a angegebene Bedeutung und ist vorzugsweise Alkyl oder Alkenyl. Besonders bevorzugt ist R'' ausgewählt aus der Gruppe bestehend aus -F, Cl, $CF_3$ und $-OCF_3$. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:

Gruppe 1: 20 bis 90 %, insbesondere 30 bis 90 %,
Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTSOH | p-Toluolsulfonsäure |

**Beispiel 1**

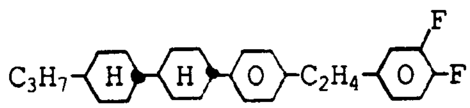

Nach Hydrierung von 1-[p-(trans-4-(trans-4-n-Propylcyclohexyl)-cyclohexyl)-phenyl]-2-(3,4-difluorphenyl)-ethen (hergestellt nach Schema 1) an Pd-C bei Raumtemperatur und üblicher Aufarbeitung erhält man 1-p-(trans-4-(trans-4-n-Propylcyclohexyl)-cyclohexyl)-phenyl)-2-(3,4-difluorphenyl)-ethan.

**Beispiel 2 bis 28:**

Analog Beispiel 1 erhält man die folgenden Verbindungen der Teilformel Ia:

| | n | X | Y | Z |
|---|---|---|---|---|
| (2) | 2 | $-CF_3$ | H | H |
| (3) | 5 | $-CF_3$ | H | H |
| (4) | 3 | $-CF_3$ | H | H |
| (5) | 5 | F | H | H |
| (6) | 2 | F | H | H |
| (7) | 3 | F | H | H |
| (8) | 5 | Cl | H | H |
| (9) | 2 | Cl | H | H |
| (10) | 3 | Cl | H | H |
| (11) | 5 | Cl | F | H |
| (12) | 2 | Cl | F | H |
| (13) | 3 | Cl | F | H |
| (14) | 5 | F | F | H |
| (15) | 2 | F | F | H |
| (16) | 4 | F | F | H |
| (17) | 2 | $-OCF_3$ | H | H |
| (18) | 3 | $-OCF_3$ | H | H |
| (19) | 5 | $-OCF_3$ | H | H |
| (20) | 2 | $-OCHF_2$ | H | H |
| (21) | 3 | $-OCHF_2$ | H | H |
| (22) | 5 | $-OCHF_2$ | H | H |
| (23) | 2 | -Cl | F | F |
| (24) | 3 | -Cl | F | F |
| (25) | 5 | -Cl | F | F |
| (26) | 2 | F | F | F |
| (27) | 3 | F | F | F |
| (28) | 5 | F | F | F |

**Beispiel 29**

Ein Gemisch von 10,5 g 1-Brom-3,4-difluorbenzol, 17,4 g 4-(trans-4-Propylcyclohexyl)-2,6-difluorstyrol (erhältlich nach Int. Patentanm. No. PCT/EP 90/02109), 0,25 g Pd-acetat, 0,6 g Tri-o-tolylphosphin, 7 g Triethylamin und 125 ml Acetonitril wird am Rückfluß erhitzt bis zur Beendigung der Reaktion. Nach üblicher Aufarbeitung erhält man trans-1-(3,4-Difluorphenyl)-2-[4-(trans-4-Propylcyclohexyl)-2,6-difluorphenyl]-ethen (K 101 N 156.5 I). Eine Lösung von 2,6 g dieser Verbindung in 30 ml Tetrahydrofuran wird mit 0,7 g Pd-C (5 %) versetzt und bei leichtem Überdruck hydriert. Man erhält 1-(3,4-Difluorphenyl)-2-[4-(trans-4-propylcyclohexyl)-2,6-difluorphenyl]-ethan.

Analog Beispiel 29 erhält man die folgenden Verbindungen der Teilformel Ig:

| n | s | X | Y | Z |
|---|---|---|---|---|
| 2 | 0 | F | H | H |
| 3 | 0 | F | H | H |
| 4 | 0 | F | H | H |
| 5 | 0 | F | H | H |

| n | s | X | Y | Z |
|---|---|---|---|---|
| 2 | 0 | F | F | H |
| 3 | 0 | F | F | H, K 66 I, Δε +8.8 |
| 4 | 0 | F | F | H |
| 5 | 0 | F | F | H |
| | | | | |
| 2 | 0 | F | F | F |
| 3 | 0 | F | F | F, K 78 I, Δε +12.0 |
| 4 | 0 | F | F | F |
| 5 | 0 | F | F | F |
| | | | | |
| 2 | 0 | $OCF_3$ | H | H |
| 3 | 0 | $OCF_3$ | H | H |
| 4 | 0 | $OCF_3$ | H | H |
| 5 | 0 | $OCF_3$ | H | H |
| | | | | |
| 2 | 0 | $OCF_3$ | F | H |
| 3 | 0 | $OCF_3$ | F | H |
| 4 | 0 | $OCF_3$ | F | H |
| 5 | 0 | $OCF_3$ | F | H |
| | | | | |
| 2 | 0 | $OCF_3$ | F | F |
| 3 | 0 | $OCF_3$ | F | F |
| 4 | 0 | $OCF_3$ | F | F |
| 5 | 0 | $OCF_3$ | F | F |

| n | s | X | Y | Z |
|---|---|---|---|---|
| 2 | 0 | $OCHF_2$ | H | H |
| 3 | 0 | $OCHF_2$ | H | H |
| 4 | 0 | $OCHF_2$ | H | H |
| 5 | 0 | $OCHF_2$ | H | H |
| 2 | 0 | $OCHF_2$ | F | H |
| 3 | 0 | $OCHF_2$ | F | H |
| 4 | 0 | $OCHF_2$ | F | H |
| 5 | 0 | $OCHF_2$ | F | H |
| 2 | 1 | F | H | H |
| 3 | 1 | F | H | H |
| 4 | 1 | F | H | H |
| 5 | 1 | F | H | H |
| 2 | 1 | F | F | H |
| 3 | 1 | F | F | H |
| 4 | 1 | F | F | H |
| 5 | 1 | F | F | H |
| 2 | 1 | F | F | F |
| 3 | 1 | F | F | F |
| 4 | 1 | F | F | F |
| 5 | 1 | F | F | F |

| n | s | X | Y | Z |
|---|---|---|---|---|
| 2 | 1 | $OCF_3$ | H | H |
| 3 | 1 | $OCF_3$ | H | H |
| 4 | 1 | $OCF_3$ | H | H |
| 5 | 1 | $OCF_3$ | H | H |
| 2 | 1 | $OCF_3$ | F | H |
| 3 | 1 | $OCF_3$ | F | H |
| 4 | 1 | $OCF_3$ | F | H |
| 5 | 1 | $OCF_3$ | F | H |
| 2 | 1 | $OCF_3$ | F | F |
| 3 | 1 | $OCF_3$ | F | F |
| 4 | 1 | $OCF_3$ | F | F |
| 5 | 1 | $OCF_3$ | F | F |
| 2 | 1 | $OCHF_2$ | H | H |
| 3 | 1 | $OCHF_2$ | H | H |
| 4 | 1 | $OCHF_2$ | H | H |
| 5 | 1 | $OCHF_2$ | H | H |
| 2 | 1 | $OCHF_2$ | F | H |
| 3 | 1 | $OCHF_2$ | F | H |
| 4 | 1 | $OCHF_2$ | F | H |
| 5 | 1 | $OCHF_2$ | F | H |

**Beispiel 30**

Eine Lösung von 0,1 m 1-(4-n-Propyl-2-fluorbiphenyl-4'-yl)-2-(3,5-difluorphenyl)-ethan (hergestellt nach WO 90/08757) und 0,1 m TMEDA in 300 ml THF wird bei ca. -65° mit 0,1 m n-BuLi (1,5 M in Hexan) tropfenweise versetzt. Man rührt noch 30 min bei dieser Temperatur und tropft hinzu bei -60 bis -70 °C 0,1 m $B(OCH_3)_3$. Man rührt noch 1/2

h nach. Dann tropft man 0,3 m $H_2O_2$ (30 %ig) zu, wobei die Temperatur +40 °C nicht übersteigen sollte. Nach extraktiver Aufarbeitung erhält man das Phenol, welches durch Kristallisation oder Destillation gereinigt werden kann.

Aus diesem Phenol erhält man $OCHF_2$-Derivat durch Einleiten von $CHClF_2$ in die THF-Lösung des Phenolats. Nach extraktiver Aufarbeitung und üblicher Reinigung erhält man 1-(4-n-Propyl-2-fluorbiphenyl-4'-yl)-2-(4-difluormethoxy-3,5-difluorphenyl)-ethan.

## Beispiel 31

In einem Autoklaven, der auf 0° gekühlt ist, füllt man 2 mol wasserfreie Flußsäure. Dann gibt man eine Mischung aus 0,18 mol Tetrachlormethan und 0,06 mol 1-(4-n-Propyl-2-fluorbiphenyl-4'-yl)-2-(4-hydroxy-3,5-difluorphenyl)-ethan (Beispiel 30) zu. Die Mischung wird ca. 8 Stunden bei 150° gerührt, abgekühlt, auf Eiswasser gegossen und mit Ether nachgewaschen. Die beiden Phasen werden ca. 30 Minuten gerührt, getrennt und die Etherlösung mit 5%iger KOH zur alkalischen Reaktion gewaschen. Nach Trocknen, Abfiltrieren, Abdestillieren und Reinigung erhält man 1-(4-n-Propyl-2-fluorbiphenyl-4'-yl)-2-(4-trifluormethoxy-3,5-difluorphenyl)-ethan.

## Beispiel 32

Eine Lösung von 0,1 m 1-(4-n-Propyl-2-fluorbiphenyl-4'-yl)-2-(3,5-difluorphenyl)-ethan (hergestellt nach Schema 3) und 0,1 m TMEDA in 300 ml THF wird bei ca. -65° mit 0,1 m n-BuLi (1,5 M in Hexan) tropfenweise versetzt. Man rührt noch 30 Min. bei dieser Temperatur und setzt dann 0,2 m N-Chlorsuccinimid in 70 ml THF langsam zu. Nach beendeter Zugabe läßt man auf -20° erwärmen und hydrolysiert mit $H_2O$. Durch Zugabe von Diethylether wird das Produkt vollständig in Lösung gebracht. Nach extraktiver Aufarbeitung und Reinigung durch Chromatographie und Kristallisation erhält man 1-(4-n-Propyl-2-fluorbiphenyl-4'-yl)-2-(4-chlor-3,5-difluorphenyl)-ethan.

## Beispiel 33

Ein Gemisch von 0,025 m CuJ, 0,0125 m $CF_3COONa$ und 0,0125 m 1-(4-n-Propyl-2-fluorbiphenyl-4'-yl)-2-(4-jod-3,5-difluorphenyl)-ethan [erhältlich gemäß Schema 15] wird in 100 ml N-Methylpyrrolidon unter Rühren auf 150° erhitzt. Nach einer Stunde arbeitet man wie üblich extraktiv auf und erhält nach chromatographischer Aufreinigung 1-(4-n-Propyl-2-fluorbiphenyl-4'-yl)-2-(4-trifluormethyl-3,5-difluorphenyl)-ethan.

## Beispiel 34

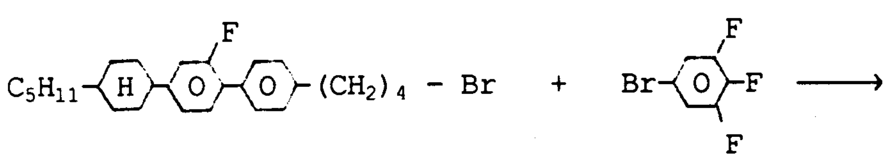

I

II

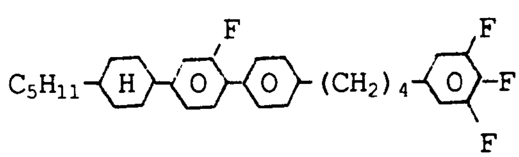

III

37,1 g I (0,1 Mol) werden in 150 ml eines Lösungsmittelgemisches von THF/Toluol (1 : 4 - Volumenverhältnis) vorgelegt, dann 11,5 g Zinkbromid wasserfrei und darauf 1,4 g Lithiumgranulat hinzugefügt. Das Gemisch wird unter Argon und Rühren 4 Std. zwischen 0 °C und 10 °C mit Ultraschall behandelt, um I in die entsprechende Dialkylzinkverbindung zu überführen. Die zinkorganische Verbindung wird mit 21,1 g II (0,1 Mol) und 1,5 g (2 mol %) 1,1'-Bis(diphenylphosphino)-ferrocen-palladium(II)dichlorid ($PdCl_2$ dppf) versetzt und nach Entfernung des Ultraschallbades und der Kühlung 24 h bei Zimmertemperatur gerührt. Es wird mit 100 ml gesättigter $NH_4Cl$-Lösung unter Rühren zersetzt, die organische Phase abgetrennt, die wäßrige Phase 2 x mit Toluol extrahiert. Die vereinigten organischen Extrakte liefern nach dem Trocknen, Einengen und Chromatografieren über Kieselgel mit Hexan III. (I ist herstellbar durch Kettenverlängerung von

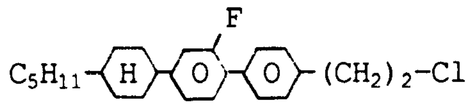

mittels Malonester.)

**Beispiel 35**

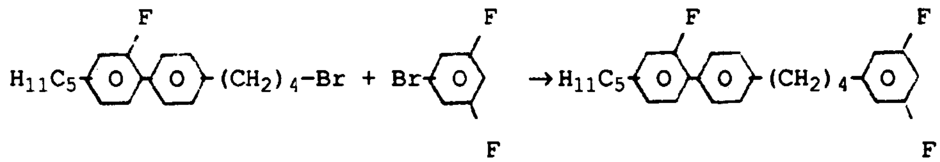

1 2 3

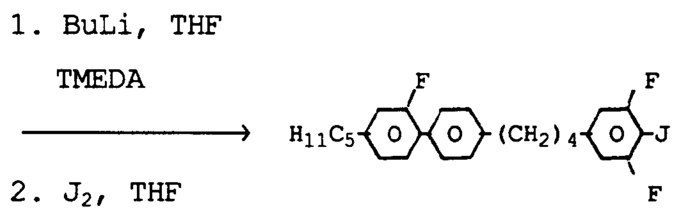


4

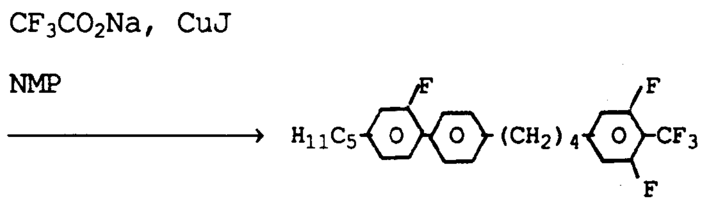


5

37,1 g (100 mmol) 1 werden analog zum Beispiel 207 durch Umsetzung mit 2 in 3 überführt.

Zu einem Gemisch aus 19,0 g (47 mmol) 3 7,5 ml TMEDA (50 mmol) und 150 ml THF werden bei -65 bis -70 °C 31 ml n-Buli (15 % in Hexan) zugetropft und es wird 1 Stunde bei -70 °C nachgerührt. Dann wird bei -65 bis -70 °C eine Lösung von 12,0 g (47 mmol) Jod in 25 ml THF zugetropft und 0,5 h bei -70 °C nachgerührt. Man erwärmt auf -30 °C, hydrolysiert mit 15 ml Wasser und reduziert überschüssiges Jod durch Zusatz von 15 ml Natriumhydrogensulfitlösung. Nach üblicher Aufarbeitung und Umkristallisation aus Hexan erhält man 23,9 g (45 mmol) 4. Aus einem Gemisch von 20,2 g (38 mmol) 4, 4,4 g (76 mmol) KF, 22,8 g (168 mmol) Natriumtrifluoracetat und 800 ml NMP werden bei 70 °C

und 4 mbar 400 ml NMP abdestilliert. Dann gibt man 14,4 g (76 mmol) getrocknetes CuJ zum Reaktionsgemisch und rührt 5 h bei 160 °C. Anschließend werden ca. 300 ml NMP abdestilliert. Man läßt auf RT abkühlen und versetzt mit 300 ml MTB-Ether. Man wäscht mit Wasser, trocknet mit $Na_2SO_4$, filtriert und engt zum Rückstand ein. Nach Chromatographie an Kieselgel mit Hexan erhält man 5.

Im folgenden sind einige besonders bevorzugte Verbindungen der Teilformel Ie angegeben:

| n | s | r | X | Y | Z |
|---|---|---|---|---|---|
| 2 | 0 | 1 | F | F | F |
| 3 | 0 | 1 | F | F | F |
| 4 | 0 | 1 | F | F | F |
| 5 | 0 | 1 | F | F | F K20N(-11)I |
| 2 | 1 | 1 | F | F | F |
| 3 | 1 | 1 | F | F | F |
| 4 | 1 | 1 | F | F | F |
| 5 | 1 | 1 | F | F | F |
| 2 | 0 | 2 | F | F | F |
| 3 | 0 | 2 | F | F | F |
| 4 | 0 | 2 | F | F | F |
| 5 | 0 | 2 | F | F | F |
| 2 | 1 | 2 | F | F | F |
| 3 | 1 | 2 | F | F | F |
| 4 | 1 | 2 | F | F | F |
| 5 | 1 | 2 | F | F | F |

sowie solche der Teilformel Ig

| n | s | r | X | Y | Z |
|---|---|---|---|---|---|
| 2 | 1 | 1 | F | H | H |
| 3 | 1 | 1 | F | H | H |
| 4 | 1 | 1 | F | H | H |
| 5 | 1 | 1 | F | H | H, K 74 N 202 I |
| 2 | 1 | 1 | $OCF_3$ | H | H |
| 3 | 1 | 1 | $OCF_3$ | H | H |
| 4 | 1 | 1 | $OCF_3$ | H | H |
| 5 | 1 | 1 | $OCF_3$ | H | H |

| | | | | | |
|---|---|---|---|---|---|
| 2 | 1 | 1 | $OCF_3$ | F | H |
| 3 | 1 | 1 | $OCF_3$ | F | H |
| 4 | 1 | 1 | $OCF_3$ | F | H |
| 5 | 1 | 1 | $OCF_3$ | F | H |
| 2 | 1 | 1 | F | F | H |
| 3 | 1 | 1 | F | F | H |
| 4 | 1 | 1 | F | F | H |
| 5 | 1 | 1 | F | F | H |
| 2 | 1 | 1 | F | F | F |
| 3 | 1 | 1 | F | F | F |
| 4 | 1 | 1 | F | F | F |
| 5 | 1 | 1 | F | F | F, K 131 N 172 I |
| 2 | 0 | 1 | F | H | H |
| 3 | 0 | 1 | F | H | H |
| 4 | 0 | 1 | F | H | H |
| 5 | 0 | 1 | F | H | H |
| 2 | 0 | 1 | F | F | H |
| 3 | 0 | 1 | F | F | H |
| 4 | 0 | 1 | F | F | H |
| 5 | 0 | 1 | F | F | H |
| 2 | 0 | 1 | F | F | F |
| 3 | 0 | 1 | F | F | F |
| 4 | 0 | 1 | F | F | F |
| 5 | 0 | 1 | F | F | F |

| | | | | | |
|---|---|---|---|---|---|
| 2 | 0 | 1 | $OCF_3$ | F | H |
| 3 | 0 | 1 | $OCF_3$ | F | H |
| 4 | 0 | 1 | $OCF_3$ | F | H |
| 5 | 0 | 1 | $OCF_3$ | F | H |
| 2 | 0 | 2 | F | H | H |
| 3 | 0 | 2 | F | H | H |
| 4 | 0 | 2 | F | H | H |
| 5 | 0 | 2 | F | H | H |
| 2 | 0 | 2 | F | F | H |
| 3 | 0 | 2 | F | F | H |
| 4 | 0 | 2 | F | F | H |
| 5 | 0 | 2 | F | F | H |
| 2 | 0 | 2 | F | F | F |
| 3 | 0 | 2 | F | F | F |
| 4 | 0 | 2 | F | F | F |
| 5 | 0 | 2 | F | F | F |

sowie solche der bevorzugten Teilformel If

| n | X | Y | Z |
|---|---|---|---|
| 2 | F | F | H |
| 3 | F | F | H |
| 4 | F | F | H |
| 5 | F | F | H |
| 2 | F | F | F |
| 3 | F | F | F |
| 4 | F | F | F |
| 5 | F | F | F |

**Patentansprüche**

1. Phenylethane der Formel I

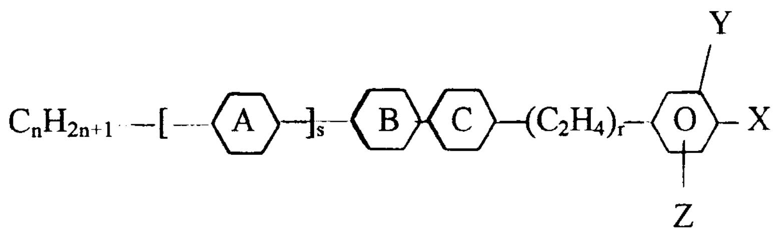

I

worin n 1 bis 7, r 1 oder 2, die Ringe A, B und C jeweils unabhängig voneinander trans-1,4-Cyclohexylen, 1,4-Phenylen oder 3-Fluor-1,4-Phenylen, Ring C auch 3,5-Difluor-1,4-phenylen, s 1 oder im Falle C = 3,5-Difluor-1,4-phenylen oder B = 3-Fluor-1,4-phenylen auch 0, X F, Cl, -CF3, -OCF3 oder -OCHF2 und Y und Z jeweils unabhängig voneinander H oder F bedeuten, mit der Maßgabe, daß im Falle

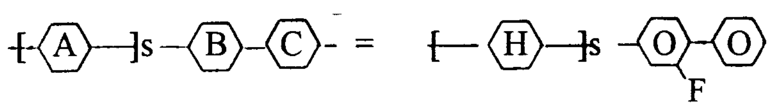

und r = 1, Y = Z = F bedeutet.

2. Phenylethane nach Anspruch 1, gekennzeichnet durch die Formel Ia

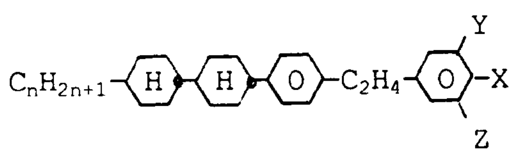

Ia

worin n, X, Y und Z die angegebene Bedeutung haben.

3. Phenylethane nach Anspruch 1, gekennzeichnet durch die Formel Ib

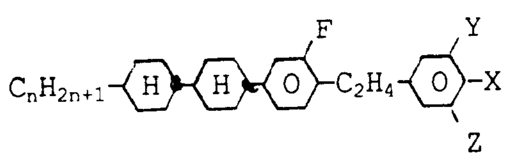

Ib

worin n, X, Y, und Z die angegebene Bedeutung haben.

4. Phenylethane nach Anspruch 1, gekennzeichnet durch die Formel Ic

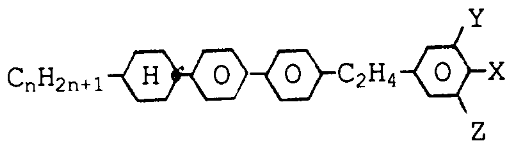

Ic

worin n, X, Y und Z die angegebene Bedeutung haben.

5. Phenylethane nach Anspruch 1, gekennzeichnet durch die Formel Id

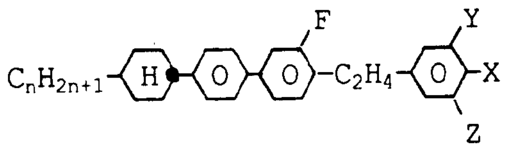

Id

worin n, X, Y und Z die angegebene Bedeutung haben.

6. Phenylethane nach Anspruch 1, gekennzeichnet durch die Formel Ie

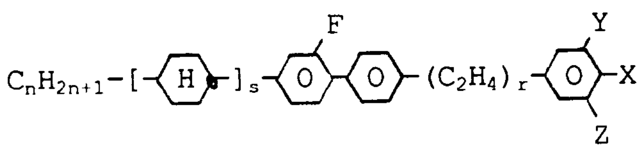

Ie

worin n, s, r, X, Y und Z die angegebene Bedeutung haben.

7. Phenylethane nach Anspruch 1, gekennzeichnet durch die Formel If

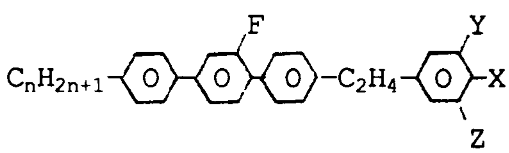

If

worin n, X, Y und Z die angegebene Bedeutung haben.

8. Phenylethane nach Anspruch 1, gekennzeichnet durch die Formel Ig

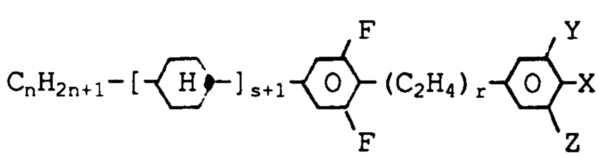

worin n, s, r, X, Y und Z die angegebenen Bedeutungen haben.

9. Verwendung der Phenylethane der Formel I nach Anspruch 1 als Komponenten flüssigkristalliner Medien für elektrooptische Anzeigen.

10. Flüssigkristallines Medium für elektrooptische Anzeigen mit mindestens zwei flüssigkristallinen Komponenten, dadurch gekennzeichnet, daß mindestens eine Komponente ein Phenylethan der Formel I nach Anspruch 1 ist.

11. Elektrooptische Anzeige auf der Basis einer Flüssigkristallzelle, dadurch gekennzeichnet, daß die Flüssigkristallzelle ein Medium nach Anspruch 10 enthält.

## Claims

1. Phenylethanes of the formula I

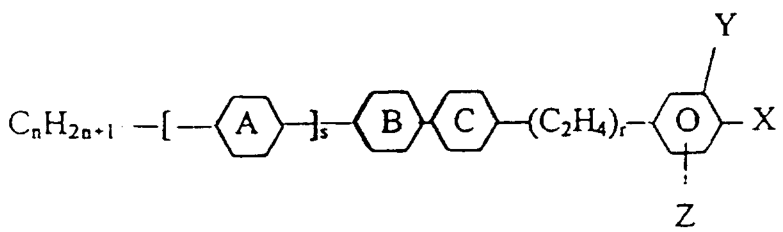

I

in which n is from 1 to 7, r is 1 or 2, the rings A, B and C are each, independently of one another, trans-1,4-cyclohexylene, 1,4-phenylene or 3-fluoro-1,4-phenylene, and ring C is alternatively 3,5-difluoro-1,4-phenylene, s is 1 or, in the case where C = 3,5-difluoro-1,4-phenylene or B = 3-fluoro-1,4-phenylene, is alternatively 0, X is F, Cl, $-CF_3$, $-OCF_3$ or $-OCHF_2$, and Y and Z are each, independently of one another, H or F, with the proviso that, in the case where

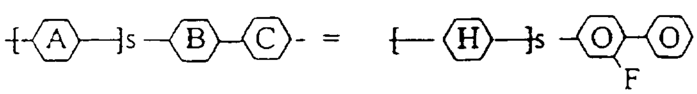

and r = 1, Y = Z = F.

2. Phenylethanes according to Claim 1, characterized by the formula Ia

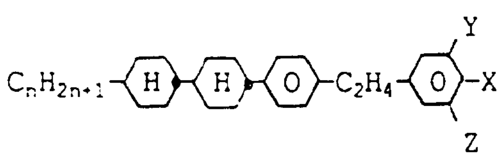

Ia

in which n, X, Y and Z are as defined above.

3. Phenylethanes according to Claim 1, characterized by the formula Ib

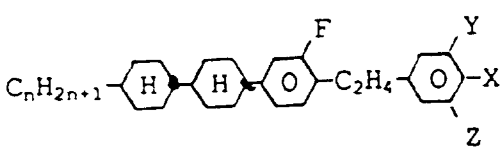

Ib

in which n, X, Y and Z are as defined above.

**4.** Phenylethanes according to Claim 1, characterized by the formula Ic

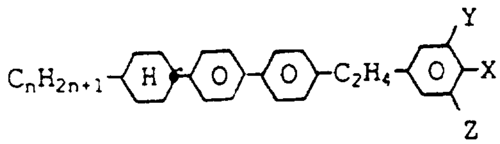

Ic

in which n, X, Y and Z are as defined above.

**5.** Phenylethanes according to Claim 1, characterized by the formula Id

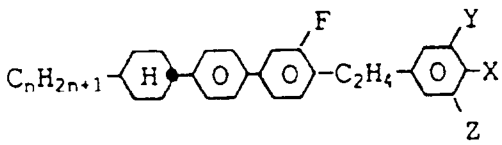

Id

in which n, X, Y and Z are as defined above.

**6.** Phenylethanes according to Claim 1, characterized by the formula Ie

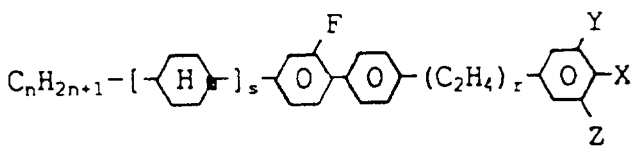

Ie

in which n, s, r, X, Y and Z are as defined above.

**7.** Phenylethanes according to Claim 1, characterized by the formula If

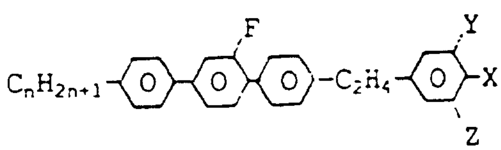

If

in which n, X, Y and Z are as defined above.

**8.** Phenylethanes according to Claim 1, characterized by the formula Ig

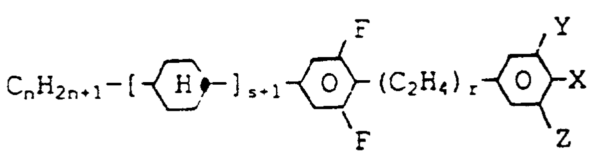

Ig

in which n, s, r, X, Y and Z are as defined above.

9. Use of the phenylethanes of the formula I according to Claim 1 as components of liquid-crystalline media for electro-optical displays.

10. Liquid-crystalline medium for electro-optical displays, having at least two liquid-crystalline components, characterized in that at least one component is a phenylethane of the formula I according to Claim 1.

11. Electro-optical display based on a liquid-crystal cell, characterized in that the liquid-crystal cell contains a medium according to Claim 10.

## Revendications

1. Phényléthanes se formule I

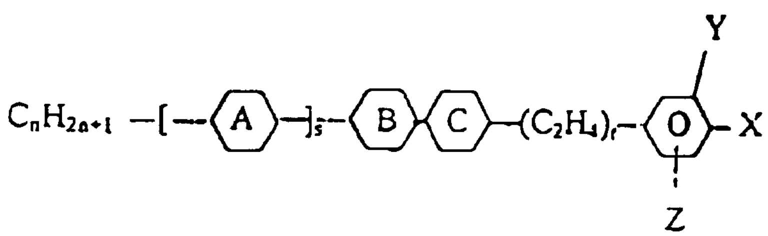

I

dans laquelle n vaut de 1 à 7, r vaut 1 ou 2, les noyaux A, B et C représentent chacun indépendamment l'un de l'autre un trans-1,4-cyclohexylène, un 1,4-phénylène ou un 3-fluoro-1,4-phénylène, le noyau C également un 3,5-difluoro-1,4-phénylène, s vaut 1, ou bien 0 dans le cas où C représente un 3,5-difluoro-1,4-phénylène ou bien où B représente un 3-fluoro-1,4-phénylène, X représente F, Cl, $-CF_3$ , $-OCF_3$ ou $-OCHF_2$ et Y et Z représentent chacun indépendamment l'un de l'autre H ou F, sous réserve que dans le cas où

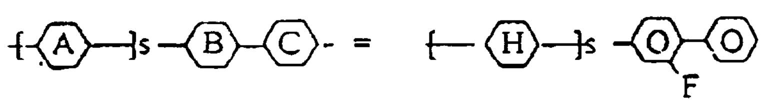

et r = 1, Y = Z = F.

2. Phényléthanes selon la revendication 1, caractérisés par la formule Ia

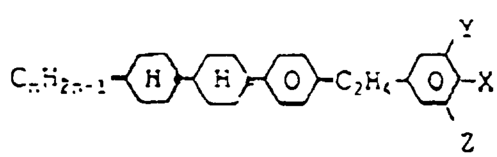

I a

dans laquelle n, X, Y et Z ont la signification donnée ci-dessus.

3. Phényléthanes selon la revendication 1, caractérisés par la formule Ib

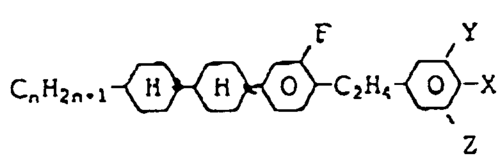

Ib

dans laquelle n, X, Y et Z ont la signification indiquée.

**4.** Phényléthanes selon la revendication 1, caractérisés par la formule Ic

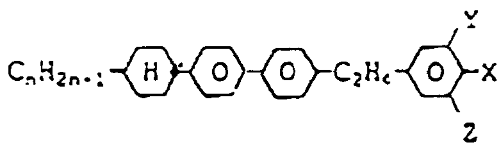

Ic

dans laquelle n, X, Y et Z ont la signification indiquée.

**5.** Phényléthanes selon la revendication 1, caractérisés par la formule Id

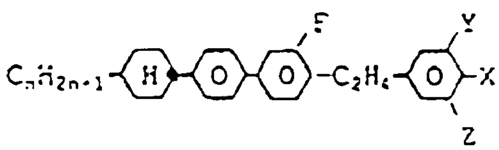

Id

dans laquelle n, X, Y et Z ont la signification indiquée.

**6.** Phényléthanes selon la revendication 1, caractérisés par la formule Ie

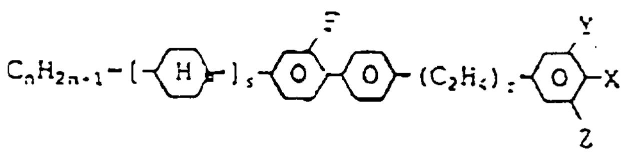

Ie

dans laquelle n, s, r, X, Y et Z ont la signification indiquée.

**7.** Phényléthanes selon la revendication 1, caractérisés par la formule If

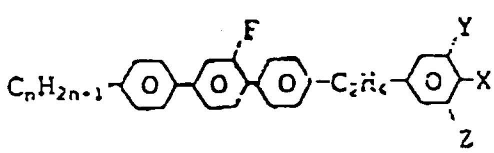

If

dans laquelle n, X, Y et Z ont la signification indiquée.

**8.** Phényléthanes selon la revendication 1, caractérisés par la formule Ig

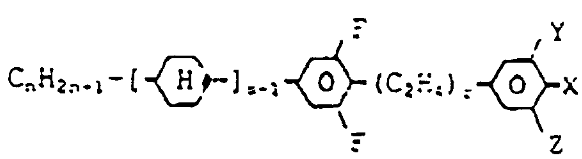

dans laquelle n, s, r, X, Y et Z ont les significations indiquées.

**9.** Application des phényléthanes de formule I selon la revendication 1 comme composants de milieux à cristaux liquides pour affichages électro-optiques.

**10.** Milieu à cristaux liquides pour affichages électro-optiques ayant au moins deux composants à cristaux liquides, caractérisé en ce qu'au moins un composant est un phényléthane de formule I selon la revendication 1.

**11.** Affichage électro-optique basé sur une cellule à cristaux liquides, caractérisé en ce que la cellule à cristaux liquides contient un milieu selon la revendication 10.